# EUROPEAN PATENT APPLICATION

(11) **EP 4 332 112 A1**
(43) Date of publication of application: **06.03.2024**
(21) Application number: 22795403.9
(22) Date of filing: 25.03.2022
(51) Int. Cl.: C07K 7/08, C12N 5/16, C12N 15/63, C12N 15/87

(54) **PEPTIDE FRAGMENT AND USE THEREOF**

(30) Priority: 28.04.2021 JP 2021075809
(71) Applicant: Toagosei Co., Ltd., Minato-ku Tokyo 105-8419 (JP)
(72) Inventor: BAILEYKOBAYASHI, Nahoko, Tsukuba-shi, Ibaraki 300-2611 (JP); YOSHIDA, Tetsuhiko, Tsukuba-shi, Ibaraki 300-2611 (JP)
(74) Representative: Bals & Vogel Patentanwälte PartGmbB
(86) International application number: PCT/JP2022/014198
(87) International publication number: WO 2022/230484

(57) **Abstract**

The present disclosure provides an artificially synthesized peptide fragment capable of efficiently introducing a target foreign substance from outside a eukaryotic cell into at least the cytoplasm of the cell. A peptide fragment disclosed herein is for introducing a target foreign substance from outside a eukaryotic cell into at least the cytoplasm of the cell, the peptide fragment being configured such that two or more of amino acid sequences VVKSLVK (SEQ ID NO: 1) are arranged in series, and another amino acid residue is not included between the amino acid sequences or a peptide linker composed of 1 to 5 amino acid residues is interposed between the amino acid sequences.

## Description

### Technical Field

The present invention relates to a method for introducing (transporting) a foreign substance from outside a eukaryotic cell into the interior of the cell, a foreign substance introduction construct including a carrier peptide fragment to be used in the method, and a peptide fragment constituting the carrier peptide fragment.

The present application claims priority based on Japanese Patent Application No. 2021-75809 filed on April 28, 2021, and the entire contents of that application are incorporated in the present description by reference.

### Background Art

Conventionally, foreign substances, such as polypeptides and especially physiologically active substances, are introduced into the cells of humans and other mammals and the like (eukaryotic cells) to transform the characteristics of the cells (and also tissues and organs composed of the cells) or to improve and enhance the functions of those cells.

For example, Patent Literature 1 discloses a foreign substance introduction construct including an amino acid sequence described in SEQ ID NO: 2 known as a nucleolar localization signal of LIM kinase 2, which is one of the protein kinases involved in intracellular signal transduction present in human endothelial cells (see Non Patent Literature 1), and a target foreign substance. Since the amino acid sequence is an excellent cell membrane penetrating peptide, the construct can pass through the cell membrane of eukaryotic cells with high efficiency. As a result, the target foreign substance can be efficiently introduced from outside a eukaryotic cell into the cytoplasm of the eukaryotic cell.

### Citation List

### Patent Literature

Patent Literature 1: WO 2011/013700
Patent Literature 2: Japanese Patent Application Publication No. 2005-330206

### Non Patent Literature

Non Patent Literature 1: JOURNAL OF BIOLOGICAL CHEMISTRY, Vol. 281, NO: 35, 2006, pp. 25223-25230

### Summary of Invention

In recent years, interest in peptides with excellent cell membrane penetrating ability has been increasing, and from the viewpoint of medicine etc., it is desirable to develop a technique for introducing foreign substances into target cells more efficiently than before.

Accordingly, the present invention was created to meet such a demand, and an object of the present invention is to provide a peptide fragment capable of efficiently introducing a target foreign substance from outside a eukaryotic cell into at least the cytoplasm of the eukaryotic cell. Another object of the present invention is to provide a foreign substance introduction construct including such a peptide fragment and the foreign substance. A further object is to provide a method for efficiently introducing such a construct from outside a eukaryotic cell into at least the cytoplasm of the cell.

In order to achieve the above objects, the present inventors searched for an amino acid sequence that has excellent cell membrane penetrating ability and is capable of introducing a foreign substance from outside a eukaryotic cell (i.e., from the outside of the cell membrane) into at least the cytoplasm of the cell, and focused attention on the amino acid sequence (VHL-related peptide motif) consisting of 15 consecutive amino acid residues from 157th to 171st of the amino acid sequence of the von Hippel-Lind (VHL) protein shown in SEQ ID NO: 3. This VHL-related peptide motif is an amino acid sequence found by the present inventors as a peptide motif involved in neural differentiation induction. As a result of intensive studies by the present inventors, it was found that excellent cell membrane penetrating ability can be achieved by repeatedly arranging in series seven amino acid residues on the C-terminal side of the VHL-related peptide motif and further substituting arginine residues with lysine residues.

That is, the peptide fragment disclosed herein is a peptide fragment for introducing a target foreign substance from outside a eukaryotic cell into at least the cytoplasm of the cell, the peptide fragment being configured such that two or more of following amino acid sequences
VVKSLUK (SEQ ID NO: 1)
are arranged in series, and another amino acid residue is not included between the amino acid sequences or a peptide linker composed of 1 to 5 amino acid residues is interposed between the amino acid sequences.

Since the peptide fragment having such a configuration has excellent cell membrane penetrating ability, this peptide fragment can efficiently introduce the target foreign substance from outside a eukaryotic cell into at least the cytoplasm of the cell.

Further, in a preferred embodiment of the peptide fragment disclosed herein, another amino acid residue is not included between the amino acid sequences. In another preferred embodiment, two amino acid sequences shown in SEQ ID NO: 1 are arranged in series. With such configurations, excellent cell membrane penetrating ability can be realized with shorter sequences.

In order to achieve the above object, in another aspect, a foreign substance introduction construct artificially produced for introducing (transporting) a target foreign substance from outside a eukaryotic cell into at least the cytoplasm of the cell is provided.

That is, the foreign substance introduction construct disclosed herein has a carrier peptide fragment composed of the peptide fragment disclosed herein, and the target foreign substance linked to the N-terminal side and/or C-terminal side of the carrier peptide fragment.

With such a configuration, a foreign substance linked to a carrier peptide fragment can be efficiently introduced into a eukaryotic cell.

Here, the term "foreign substance" is inclusive of inorganic compounds and organic compounds capable of linking directly or indirectly, via a suitable linker, to the N-terminal side or C-terminal side of the carrier peptide fragment, and has a molecular size and chemical properties that allow it to be introduced into a eukaryotic cell.

In a preferred embodiment of the foreign substance introduction construct disclosed herein, the foreign substance is any organic compound selected from the group consisting of polypeptides, nucleic acids, dyes, and drugs.

Here, the term "polypeptide" refers to a polymer having a structure in which a plurality of amino acids is linked together by peptide bonds. The polypeptide is not limited by the number of peptide bonds (that is, the number of amino acid residues). That is, the polypeptides include those commonly called peptides composed of about 10 or more but fewer than 300 amino acid residues, and those commonly called proteins (high-molecular-weight compounds typically composed of 300 or more amino acid residues). Polypeptides and proteins are not strictly distinguished in the art. In the present description, polymers (including oligomers) consisting of a plurality of amino acid residues are collectively referred to herein as polypeptides.

Further, the term "nucleic acid" refers to a polymer of nucleotides, and encompasses DNA and RNA. The "nucleic acid" is not limited by the number of bases.

Also, preferably, the foreign substance is a mature polypeptide derived from any species of organism or precursor polypeptide thereof, and is a synthetic polypeptide having an amino acid sequence corresponding to the mature polypeptide or precursor polypeptide thereof as the foreign substance, together with the amino acid sequence of the carrier peptide fragment.

Further, more preferably, the amino acid sequence corresponding to the mature polypeptide or precursor polypeptide thereof as the foreign substance is arranged on the N-terminal side of the carrier peptide fragment.

Furthermore, in order to achieve the above objects, the technique disclosed herein provides a method for efficiently introducing (transporting) a target foreign substance from outside a eukaryotic cell into at least the cytoplasm of the cell. That is, the foreign substance introduction method disclosed herein includes:
(1) a step of preparing a foreign substance introduction construct disclosed herein;
(2) a step of supplying the foreign substance introduction construct to a sample including a target eukaryotic cell; and
(3) a step of incubating the sample, to which with the foreign substance introduction construct has been supplied, to thereby introduce the foreign substance introduction construct into the eukaryotic cell in the sample.

According to the method for introducing a foreign substance having the above configuration, a foreign substance introduction construct constructed by linking a target foreign substance (typically an organic compound such as a polypeptide, nucleic acid, dye, drug, etc.) directly or indirectly, via a suitable linker, to the N-terminal side and/or C-terminal side of the carrier peptide fragment can be supplied to a sample including a eukaryotic cell of interest (typically a culture containing the cell) (that is, added to an existing eukaryotic cells), thereby making it possible to introduce the target foreign substance efficiently into the cytoplasm from outside the eukaryotic cell (outside the cell membrane) through the cell membrane.

In a preferred embodiment of the foreign substance introduction method disclosed herein, the foreign substance is characterized by being any organic compound selected from the group consisting of polypeptides, nucleic acids, dyes and drugs. A construct produced to include this kind of organic compound may be efficiently introduced into the target cell.

In another preferred embodiment of the foreign substance introduction method disclosed herein, the foreign substance is a mature polypeptide derived from any species of organism or precursor polypeptide thereof, and the foreign substance introduction construct is a synthetic polypeptide having an amino acid sequence corresponding to a mature polypeptide or precursor polypeptide thereof as the foreign substance, together with the amino acid sequence of the carrier peptide fragment.

With such a configuration, the synthetic peptide having the amino acid sequence of the mature polypeptide or precursor polypeptide thereof and the amino acid sequence of the carrier peptide fragment is efficiently introduced into the target eukaryotic cell.

In another preferred embodiment of the foreign substance introduction method disclosed herein, the amino acid sequence corresponding to the mature polypeptide or precursor polypeptide thereof as the foreign substance is arranged on the N-terminal side of the carrier peptide fragment.

With such a configuration, the amino acid sequence of the mature polypeptide or precursor polypeptide thereof is more efficiently introduced into the target eukaryotic cell.

In another preferred embodiment of the foreign substance introduction method disclosed herein, the eukaryotic cell of interest into which the foreign substance introduction construct is to be introduced is a human or non-human mammalian cell.

With such a configuration, a foreign substance is efficiently introduced into the cytoplasm of a human or non-human mammalian cell.

### Brief Description of Drawings

[Fig. 1]

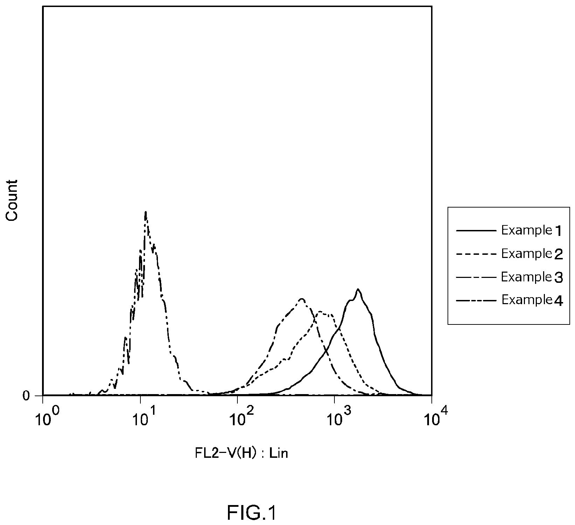

Fig. 1 is a histogram showing the relationship between fluorescence intensity and cell count obtained by flow cytometer analysis of cells after the addition of a construct in a test (Example 1) in which a construct (Sample 1) having the amino acid sequence shown in SEQ ID NO: 4 and FAM was added to a HeLa cell culture medium, a test (Example 2) in which a construct (Sample 2) having the amino acid sequence shown in SEQ ID NO: 5 and FAM was added, a test (Example 3) in which a construct (Sample 3) having the amino acid sequence shown in SEQ ID NO: 6 and FAM was added, and a test (Example 4) in which FAM was added. The X-axis (horizontal axis) indicates fluorescence intensity, and the Y-axis (vertical axis) indicates cell count.

### Description of Embodiments

Preferred embodiments of the technique disclosed herein will be described hereinbelow. Matters other than those specifically mentioned in this description that are necessary for implementation (for example, methods for chemical synthesis of peptides, cell culture methods, and general matters such that relate to the preparation of compositions containing peptides and nucleic acids as components) ) can be understood as design items by a person skilled in the art based on the related art in fields such as cell engineering, physiology, medicine, pharmacy, organic chemistry, biochemistry, genetic engineering, protein engineering, molecular biology, and genetics.

In addition, the technique disclosed herein can be implemented based on the contents disclosed in this description and common technical knowledge in the relevant field. In the following explanation, amino acids are sometimes represented by one-letter notation (threeletter notation in sequence listings) conforming to the nomenclature for amino acids shown in the IUPAC-IUB guidelines. In the present description, the term "amino acid residue" is inclusive of the N-terminal amino acid and C-terminal amino acid of a peptide chain, unless otherwise specified.

In the present description, the term "synthetic peptide" means a peptide fragment that has been manufactured by artificial chemical synthesis or biosynthesis (that is, production based on genetic engineering) and can exist stably in a given composition, rather than a peptide chain that exist stably by itself in nature. Here, "peptide" is a term that refers to an amino acid polymer having a plurality of peptide bonds and is not limited by the number of amino acid residues.

In the amino acid sequences described in this description, the left side always represents the N-terminal side and the right side always represents the C-terminal side. Further, in the present specification, when the numerical range is described as A to B (where A and B are arbitrary numerical values), it is the same as the general interpretation, and means A or more and B or less, and ranges above A and below B are included.

The peptide fragment disclosed herein includes a plurality of (two or more) amino acid sequences: VVKSLVK shown in SEQ ID NO: 1. Further, the amino acid sequences shown in SEQ ID NO: 1 are arranged in series.

The amino acid sequence shown in SEQ ID NO: 1 is the sequence in which a lysine residue is substituted for the arginine residue which is third from the N terminal among the 7, 165th to 171st, amino acid residues from N terminal of the amino acid sequence constituting the von Hippel-Lind (VHL) protein, which is known to be expressed in nerve cells of the central nervous system. The VHL protein is known to have a VHL-related peptide motif (amino acid sequence composed of a total of 15, 157th to 171st, amino acid residues from the N terminal of the VHL protein (SEQ ID NO: 3)) exhibiting neuronal differentiation-inducing properties (see Patent Literature 2). Although the VHL-related peptide motif has the cell membrane penetrating ability, the penetration efficiency is not high. However, the study conducted by the present inventors has shown that a peptide fragment including a repeating sequence of 7 amino acid residues on the C-terminal side of the VHL-related motif has excellent cell membrane penetrating ability. Furthermore, substitution of lysine residues for arginine residues in such repetitive sequences was found to significantly improve cell membrane penetrating ability beyond the range predictable for conservative amino acid replacement (so-called conservative replacement).

In the peptide fragment disclosed herein, two or more amino acid sequences shown in SEQ ID NO: 1 are arranged in series. The number to be arranged may be, for example, 3 or more, or 4 or more. Although not particularly limited, the number of amino acid sequences shown in SEQ ID NO: 1 that are arranged in series may be, for example, 10 or less, and may be 5 or less. In the present description, "n (n is a natural number of 2 or more) amino acid sequences shown in SEQ ID NO: 1 are arranged" means that the amino acid sequence consisting of seven amino acid residues shown in SEQ ID NO: 1 is taken as one unit, and n units are arranged.

A plurality of amino acid sequences shown in SEQ ID NO: 1 that is contained in a peptide fragment may be directly (contiguously) linked to each other without containing other amino acid residues or may be indirectly linked via a peptide linker. The number of amino acid residues constituting this peptide linker is not particularly limited as long as the cell membrane penetrating ability is not significantly impaired, but may be, for example, 1 to 5, preferably 1 to 3. In addition, any type of amino acid residue may be included to constitute the peptide linker as long as the cell membrane penetrating ability is not significantly impaired. Among them, preferred examples include amino acids that have uncharged side chains and are less likely to cause steric hindrance. Examples of such amino acids include glycine, alanine, serine and the like, and it is preferable to select one or two or more of these to form a peptide linker.

In addition, the peptide fragment may contain other amino acid residues at the N-terminal and/or C-terminal end as long as the cell membrane penetrating ability is not significantly impaired. The number of other amino acid residues is not particularly limited, but may be, for example, 5 or less, preferably 3 or less, at each end. The type of other amino acids is not particularly limited, but may be, for example, amino acid residues that have uncharged side chains and are less likely to cause steric hindrance, such as glycine, alanine, serine, and the like.

When the total number of amino acid residues constituting the peptide fragment is 100%, the ratio of the amino acid sequence shown in SEQ ID NO: 1 is preferably 70% or more, more preferably 80% or more, further preferably 90% or more, particularly preferably 95% or more, and may be 100% (that is, composed only of repeats of the amino acid sequence shown in SEQ ID NO: 1). The higher the ratio of the amino acid sequence shown in SEQ ID NO: 1, the more effectively the cell membrane penetrating ability can be exhibited.

The total number of amino acid residues constituting the peptide fragment is not particularly limited, but is, for example, 50 or less, and can be 40 or less, 30 or less, 25 or less, 20 or less, or 15 or less.

The peptide fragment disclosed herein may contain a modified sequence of the amino acid sequence shown in SEQ ID NO: 1 within a range in which cell membrane penetrating ability is not impaired. Here, the term "modified sequence" refers to an amino acid sequence formed by replacement, deletion and/or addition (insertion) of one or several (for example, two or three) amino acid residues (modified amino acid sequence). Examples of modified sequences of the technique disclosed herein include sequences generated by so-called conservative replacement (conservative amino acid replacement) in which of the total number of amino acid residues constituting the amino acid sequence shown in SEQ ID NO: 1 contained in the peptide fragment (for example, when two amino acid sequences shown in SEQ ID NO: 1 are included, a total of 14 amino acid residues), 1 or 2 amino acid residues are conservatively replaced, sequences obtained by addition (insertion) or deletion of 1, 2 or 3 amino acid residues, etc. Such a slightly modified sequence can be easily used by a person skilled in the art on the basis of the information disclosed herein, and therefore it is included in the technical concept of the "peptide fragment (carrier peptide fragment)" disclosed herein.

Specific examples of conservative replacement include, for example, sequences obtained by substituting a hydrophobic amino acid residue for another hydrophobic amino acid residue (for example, mutual replacement of leucine residue, isoleucine residue and valine residue).

The foreign substance introduction construct disclosed herein has a carrier peptide fragment composed of the peptide fragment disclosed herein and the target foreign substance linked to the N-terminal side and/or C-terminal side of the carrier peptide fragment.

The "carrier peptide fragment" disclosed herein is a sequence defined (ascertained) by the amino acid sequence of the peptide fragment disclosed herein, and is an amino acid sequence that exhibits cell membrane penetrating ability in a eukaryotic cell. Therefore, the foreign substance introduction construct having such a carrier peptide fragment and a target foreign substance linked to the N-terminal side and/or the C-terminal side of the carrier peptide fragment can be introduced from outside a eukaryotic cell into at least the cytoplasm of the cell with high efficiency.

The foreign substance introduction construct can be designed and constructed by linking (binding) a desired foreign substance directly or indirectly, via an appropriate linker, to the N-terminal side and/or C-terminal side of the carrier peptide fragment.

The linker is not particularly limited, and may be a peptide linker or a non-peptide linker. Although not particularly limited, the amino acid sequence that constitutes the peptide linker is preferably a flexible amino acid sequence that does not cause steric hindrance. The peptide linker contains 10 or less (more preferably 1 to 5, for example 1, 2, 3, 4 or 5) amino acid residues including one or two or more amino acid residues selected from glycine, alanine, serine and the like. β-Alanine may also be employed as a component of such linkers. The non-peptide linker is not particularly limited, but may be, for example, an alkyl linker, a PEG (polyethylene glycol) linker, an aminohexanoyl spacer, or the like.

The foreign substance is typically an organic compound such as a polypeptide, nucleic acid, dye, drug or the like.

The foreign substance can be, for example, a polypeptide. When the foreign substance is a polypeptide, the target foreign substance introduction construct can be produced by designing a peptide chain that includes the amino acid sequence constituting this polypeptide and the amino acid sequence constituting the carrier peptide fragment, and biosynthesize or chemically synthesize the peptide chain. The foreign substance introduction construct can also be constructed by using various conventionally known scientific methods to link an organic compound that functions as a nucleic acid, such as various kinds of DNA and RNA, a dye (such as various fluorescent dyes including FAM and FITC) or a drug (for example, an antitumor drug containing a nucleic acid antitumor agent such as 5-fluorouracil (5FU), or an antiviral drug such as azidothymidine (AZT)) either directly or indirectly to the N-terminal side and/or C-terminal side of the carrier peptide fragment described above.

Although not particularly limited, the function of foreign substance include, for example, promotion of stem cell differentiation induction (stem cell differentiation inducing activity), suppression of tumor cell proliferation (antitumor activity), suppression of virus-infected cell proliferation (antiviral activity), and the like.

In the foreign substance introduction construct, the number of foreign substances linked to the carrier peptide fragment is not particularly limited. That is, one or two or more foreign substances may be linked to one carrier peptide fragment. Although not particularly limited, for example, a polypeptide, nucleic acid, drug, or the like may be linked to the N-terminal side of one carrier peptide fragment, and a dye may be linked to the C-terminal side. Binding a dye to the carrier peptide fragment is preferred because it facilitates evaluation of the efficiency of introduction of the foreign substance introduction construct into the eukaryotic cell and also evaluation of intracellular localization.

In addition, when the foreign substance is a polypeptide, the polypeptide (amino acid sequence) to be used is not particularly limited. For example, substances with a relatively large number of amino acid residues, such as polypeptides or proteins with about 100 to 1000 amino acid residues, can also be used as foreign substances.

Typically, the total number of amino acid residues constituting a synthetic peptide prepared as a foreign substance introduction construct is several to several tens (such as 10) or more, and is suitably 1000 or less, or preferably 600 or less, more preferably 500 or less, and particularly preferably 300 or less (such as 10 to 300). Polypeptides of such length are easy to synthesize (biosynthesis, chemical synthesis) and easy to use.

A mature form or a precursor (including pro-form and prepro-form) of a polypeptide involved in functions such as the development, differentiation, growth, carcinogenesis, homeostasis and metabolic regulation of various cells and tissues (organs) is preferred as the foreign substance. Further, the foreign substance introduction method disclosed herein can also be performed to introduce a polypeptide with a previously unknown function into cells and elucidate the function of this polypeptide in cells (biological tissue).

For example, when the eukaryotic cell of interest into which the foreign substance is to be introduced is a stem cell of a human or other mammalian animal, it is preferable to use a mature form or precursor of a polypeptide having various physiological functions associated with differentiation induction of the stem cell. The term "stem cell" encompasses somatic stem cells, embryonic stem cells and induced pluripotent stem cells (hereunder called iPS cells). Further, when the eukaryotic cell into which the foreign substance is to be introduced is a cancer cell (tumor cell), it is desirable to use various polypeptides associated with apoptosis induction in that cancer cell (tumor cell). Alternatively, in this case it is also preferable to use a polypeptide capable of preventing the cancer cell (tumor cell) from suppressing the function of the immune monitoring mechanism. Furthermore, when the eukaryotic cell which is the target for introduction is a bacteria-infected cell or virus-infected cell, it is preferable to use various polypeptides associated with apoptosis induction in such infected cells, or polypeptides capable of suppressing the proliferation of bacteria or viruses in such infected cells, or polypeptides capable of preventing bacterial or viral infection from spreading from such infected cells.

Similarly to the peptide fragment, the polypeptide that is the foreign substance may include modified amino acid sequences formed by substitution, deletion and/or addition (insertion) of one or more amino acid residues as long as the function of the polypeptide is retained.

The foreign substance introduction construct is preferably one in which at least one amino acid residue has been amidated. The structural stability (such as protease resistance) of the foreign substance introduction construct in the cytoplasm and in the nucleus can be improved by amidating a carboxyl group of an amino acid residue (typically the C-terminal amino acid residue of the peptide chain).

For example, when the foreign substance is linked to the N-terminal side of the carrier peptide fragment, it is preferable to amidate the C-terminal amino acid residue of the carrier peptide fragment. Further, when the foreign substance is, for example, a polypeptide and this polypeptide is linked to the C-terminal side of the carrier peptide fragment, it is preferable to amidate the C-terminal amino acid residue of the polypeptide.

Those of the peptide fragments and foreign substance introduction constructs disclosed herein that have relatively short peptide chains (including a polypeptide constructed as a foreign substance, a carrier peptide fragment and a peptide linker) can be easily constructed by ordinary chemical synthesis methods. For example, either a conventionally known solid-phase synthesis method or liquid-phase synthesis method may be adopted. A solid phase synthesis method employing Boc (t-butyloxycarbonyl) or Fmoc (9-fluorenylmethoxycarbonyl) as a protecting group for amino groups is preferred. That is, the abovementioned peptide chain having the desired amino acid sequence and modifications (C-terminal amidation etc.) can be synthesized by solid-phase synthesis using a commercial peptide synthesizer. It is possible to synthesize only part of the peptide chain by these methods, for example, only the carrier peptide fragment, or a peptide chain that includes the carrier peptide fragment and a peptide linker part can be synthesized.

Alternatively, a peptide part may be synthesized by biosynthesis based on genetic engineering methods. That is, a polynucleotide (typically DNA) is synthesized having a nucleotide sequence (including ATG initiation codon) coding for the desired amino acid sequence. A recombinant vector having a gene construct for expression composed of the synthesized polynucleotide (DNA) together with various regulatory elements (including a promoter, ribosome linking site, terminator, enhancer, and various cis-elements that control the expression level) for expressing the amino acid sequence in host cells is then constructed according to the host cells.

This recombinant vector is then introduced into predetermined host cells (such as yeast, insect, or plant cells) by ordinary techniques, and the host cells or a tissue or organism containing the host cells is cultured under predetermined conditions. The target peptide can thus be produced in cells. The peptide part can then be isolated from the host cells (or from medium if it has been excreted), and refolded, purified and the like as necessary to obtain the target peptide part.

Methods that have been conventionally used in the field may be adopted, as they are, as the methods for constructing the recombinant vector, methods for introducing the constructed recombinant vector into host cells, and the like, and detailed explanations are omitted because these methods themselves are not a particular feature of the present technique.

For example, a fusion protein expression system can be used for efficient largevolume production in host cells. That is, a gene (DNA) coding for the amino acid sequence of the target polypeptide is chemically synthesized, and this synthetic gene is introduced into a suitable site of an appropriate fusion protein expression vector (for example, the pET series provided by Novagen or a GST (glutathione S-transferase) fusion protein expression vector such as the pGEX series provided by Amersham Biosciences). Host cells (typically E. coli) are then transformed with this vector. The resulting transformant is cultured to prepare the target fusion protein. Next, this protein is extracted and purified. The resulting purified fusion protein is then cleaved with a predetermined enzyme (protease), and the released target peptide fragment (that is, the designed artificial polypeptide) is collected by a method such as affinity chromatography. The target foreign substance introduction construct (artificial polypeptide) can by constructed using such a conventionally known fusion protein expression system (using for example a GST/His system provided by Amersham Biosciences).

Alternatively, template DNA for a cell-free protein synthesis system (that is, a synthetic gene fragment including a nucleotide sequence coding for the amino acid sequence of the peptide part of the foreign substance introduction construct) can be constructed, and the target polypeptide can be synthesized in vitro with a so-called cell-free protein synthesis system using various compounds (ATP, RNA polymerase, amino acids, and the like) necessary for synthesizing the peptide part. See, for example, the report by Shimizu et al. (Shimizu et al., Nature Biotechnology, 19, 751-755 (2001)) or Madin et al (Madin et al., Proc. Natl. Acad. Sci. USA, 97(2), 559-564 (2000)) with respect to cell-free protein synthesis systems. At the time of filing of this application, many companies are already engaged in contract production of polypeptides based on the techniques described in these publications, and cell-free protein synthesis kits are commercially available (for example, from Cell Free Sciences Co., Ltd. in Japan).

A single-stranded or double-stranded polynucleotide including a nucleotide sequence coding for the peptide part of the foreign substance introduction construct and/or a nucleotide sequence complementary to that sequence, can be easily manufactured (synthesized) by conventionally known methods. That is, by selecting codons corresponding to each amino acid residue constituting the designed amino acid sequence a nucleotide sequence corresponding to that amino acid sequence can be easily determined and provided. Once the nucleotide sequence has been determined, a (single-stranded) polynucleotide corresponding to the desired nucleotide sequence can be easily obtained with a DNA synthesizer or the like. The resulting single-stranded DNA can then be used as a template to obtain target double-stranded DNA by using various enzymatic synthesis methods (typically PCR). The polynucleotide may be in the form of either DNA or RNA (mRNA or the like). The DNA may be provided as either a double or single stranded. When it is a single strand, it may be a coding strand (sense strand) or a non-coding strand (antisense strand) complementary to the coding strand.

The resulting polynucleotide can then be used as a material for constructing a recombinant gene (expression cassette) for peptide production in various host cells or in a cell-free protein synthesis system as described above.

The foreign substance introduction construct may be used advantageously as an active component of a composition for use based on the function of the foreign substance. The foreign substance introduction construct may also be in the form of a salt so long as the function of the foreign substance is not impaired. For example, the foreign substance introduction construct can be used in the form of an acid-addition salt, which can be obtained by adding and reacting a commonly used inorganic or organic acid in ordinary methods. Hence, the "foreign substance introduction construct" described in this description and set forth in the claims is inclusive of such salt forms.

The foreign substance introduction construct may also be provided as a composition that may contain various medically (pharmacologically) acceptable carriers suited to the mode of use in addition to the foreign substance introduction construct as an active component.

These carriers are preferably carriers commonly used, for example, as diluents, excipients, and the like in peptide drugs. These carriers may differ appropriately according to the use and form of the foreign substance introduction construct, but typical examples include water, physiological buffers, and various organic solvents. An aqueous alcohol (such as ethanol) solution of a suitable concentration, glycerol, or a non-drying oil such as olive oil or a liposome for example may also be used as a carrier. Examples of accessory components that may be included in medical compositions include various fillers, bulking agents, binders, humectants, surfactants, dyes, perfumes and the like.

The form of the composition is not particularly limited. For example, typical forms include liquids, suspensions, emulsions, aerosols, foams, granules, powders, tablets, capsules, ointments and the like. For using injection or the like, the composition may also be in the form of a freeze-dried product or granules that are dissolved in saline or a suitable buffer (such as PBS) or the like immediately before use to prepare a drug solution.

The processes for preparing drugs (compositions) of various forms from the foreign substance introduction construct (principal component) and various carriers (accessory components) may, in themselves, conform to conventionally known methods, and detailed explanations are omitted because these preparation methods themselves are not a feature of the disclosure. Detailed information about formulations is described for example in Comprehensive Medicinal Chemistry, Corwin Hansch Ed., Pergamon Press (1990).

A method for introducing the foreign substance introduction construct either in vivo or in vitro by using the foreign substance introduction construct (composition) is provided. This method generally includes the following steps (1) to (3):
(1) a step of preparing a foreign substance introduction construct discloses herein;
(2) a step of supplying the foreign substance introduction construct to a sample containing a target eukaryotic cell, and
(3) a step of incubating the sample to which the foreign substance introduction construct has been supplied to thereby introduce the construct into the eukaryotic cell in the sample.

In vivo, the "eukaryotic cell" may encompass for example various tissues, organs, blood, lymph and the like. In vitro, the "eukaryotic cell" may encompass various cell masses, tissues, organs, blood and lymph extracted from living organisms, as well as cell lines and the like.

A composition including the construct disclosed above can be used in vivo by a method and in a dosage suited to the form and object of the composition. For example, in liquid form it may be administered by intravenous, intramuscular, subdermal, intradermal, or intraperitoneal injection in the desired dose to an affected area (for example, a malignant tumor tissue, virus-infected tissue, inflamed tissue, and the like) of a patient (that is, a living organism). Alternatively, in a solid form such as a tablet or a gel or aqueous jelly form such as an ointment, the composition can also be administered directly to a predetermined tissue (that is, an affected area such as a tissue or organ containing tumor cells, virus-infected cells, inflamed cells or the like). It can also be administered orally in a solid form such as a tablet. In the case of oral administration, it is preferable to use a capsule or protective material (coating) to prevent degradation by digestive enzymes in the digestive tract.

In the case of eukaryotic cells cultured in vitro, a suitable amount of the composition disclosed herein (that is, a suitable amount of the foreign substance introduction construct) may also be supplied at least once to a culture solution of the target eukaryotic cells. The amount supplied each time and the number of supply cycles is not particularly limited because they may differ depending on conditions such as the type of eukaryotic cell being cultured, the cell density (cell density at beginning of culture), number of passages, culture conditions, type of medium, and the like. For example, the composition is preferably added once, twice, or more than twice so that the concentration of the carrier peptide fragment in the culture solution is within a range about 0.05 µM to 100 µM, for example, within a range about 0.5 µM to 50 µM, or for example, within a range about 1 µM to 20 µM for example.

One example of an introduction method in vitro is given in the examples below.

A method of evaluating the introduction efficiency of the foreign substance introduction construct is not particularly limited. For example, when a dye (typically a fluorescent dye compound) is linked to the construct, the introduction efficiency into eukaryotic cells can be evaluated by microscope observation (such as fluorescent microscope observation), flow cytometry or the like. The introduction efficiency of the construct can also be evaluated by immunochemical methods (such as Western blot or immune cell staining) using an antibody that specifically recognizes the peptide part of the construct.

Some examples of the technique disclosed herein are explained below, but not with the intention of limiting the technique disclosed herein to what is shown in these examples.

### <Preparation of Foreign Substance Introduction Construct>

Three kinds of synthetic peptides (peptides 1 to 3) shown in Table 1 were prepared. Peptide 1 is a peptide fragment composed of the amino acid sequence shown in SEQ ID NO: 4, and is configured of an amino acid sequence in which two amino acid sequences shown in SEQ ID NO: 1 are directly linked in series. Peptide 2 is a peptide fragment composed of an amino acid sequence (SEQ ID NO: 5) in which two sets of 7 amino acid residues on the C-terminal side of the VHL-related motif are directly linked in series. Peptide 3 is a peptide fragment composed of an amino acid sequence (SEQ ID NO: 6) in which two lysine residues included in the amino acid sequence shown in SEQ ID NO: 5 are each substituted with an arginine residue.

All peptides 1 to 3 were synthesized by solid-phase synthesis (Fmoc method) using a commercially available peptide synthesizer according to the manual. Peptides 1 to 3 were all synthesized by amidating (-CONH₂) the carboxyl group (-COOH) of the C-terminal amino acid residue.

Since the mode of use of the peptide synthesizer does not by itself characterize the technique disclosed herein, detailed description is omitted.

### [Table 1]

**Table 1**

| Peptide No. | Amino acid sequence | SEC ID |
|---|---|---|
| 1 | VVKSLVKVVKSLVK | 4 |
| 2 | VVRSLVKVVRSLVK | 5 |
| 3 | VVRSLVRVVRSLVR | 6 |

Next, the fluorescent dye FAM (C₂₁H₁₂O₇: 5(6)-carboxyfluorescein, molecular weight 376.3, excitation wavelength 495 nm, fluorescent wavelength 520 nm) was linked directly as a foreign substance by an ordinary method to the amino acid residues at the N-terminals of the peptides 1 to 3 to prepare foreign substance introduction constructs ("Samples 1 to 3"). Samples 1 to 3 were each diluted with DMSO to prepare sample solutions 1 to 3 with a concentration of 2 mM.

### <Evaluation of Cell Membrane Penetrating Ability of Samples 1 to 3>

The cell membrane penetrating ability of samples 1 to 3 was evaluated using HeLa cells (an established cell line derived from human cervical cancer cells) as eukaryotic cells. As shown in Table 2, the test in which sample solution 1 was added to the HeLa cell culture medium was defined as Example 1, the test in which sample solution 2 was added was defined as Example 2, the test in which sample solution 3 was added was Example 3, and the test in which the FAM solution diluted with DMSO was added was defined as Example 4. In addition, "FAM" in Table 2 indicates the abovementioned fluorescent dye.

### [Table 2]

**Table 2**

| | Configuration of foreign substance introduction construct |
|---|---|
| Example 1 | FAM-VVKSLVKVVKSLVK-CONH₂ |
| Example 2 | FAM-VVRSLVKVVRSLVK-CONH₂ |
| Example 3 | FAM-VVRSLVRVVRSLVR-CONH₂ |
| Example 4 | FAM |

### (Example 1)

HeLa cells were cultured in DMEM (Dulbecco's modified Eagle's medium, manufactured by Fuji Film Wako Pure Chemical Industries, Cat. No. 043-30085) containing 10% FBS (fetal bovine serum), which is an ordinary culture medium.

HeLa cells adhering to the culture plate were washed with PBS, 0.25% trypsin/EDTA solution was added, and the cells were incubated for 3 min at 37°C. After incubation, the same DMEM medium including 10% FBS was added to deactivate the trypsin, and the cells were then precipitated by centrifugation for 5 min at 150×g. The supernatant produced by centrifugation was removed, and the same DMEM including 10% FBS was added to the precipitate (cell pellet) to prepare an approximately 1 × 10⁵ cells/mL cell suspension. A total of 2 mL of the cell suspension was added to the wells of a commercial 6-hole (well) plate (AGC Techno Glass Co., Ltd) to seed the cells (at approximately 2 × 10⁵ cells/well). Subsequent culturing for 3 h at 37°C under conditions of 5% CO₂ caused the cells to adhere to the bottom of the wells.

Next, the above 2 mM sample solution 1 was diluted with DMEM including 10% FBS to prepare a sample solution 1 in which the concentration of Sample 1 was 20 µM. A total of 1 mL of the culture supernatant was removed from the wells after the 3 h of culture, and 1 mL of the 20 µM sample solution 1 was added to the wells (that is, the concentration of Sample 1 in the culture solution in the wells was made 10 µM and the DMSO concentration was made 0.5%). The cells were then incubated for 20 h at 37°C under conditions of 5% CO₂. After such 20-h incubation, the culture supernatant was removed from the wells, and the cells in the wells were washed twice with 1 mL of PBS. A total of 200 µL of 0.25% trypsin/EDTA solution was then added to the wells, and the cells were incubated for 3 min at 37°C. After this incubation, 400 µL of the above DMEM containing 10% FBS was added to the wells to deactivate the trypsin, the cell suspension in the well was thereafter transferred to a tube, and the cells were collected. A total of 600 µL of PBS was then added to the wells to wash the wells. The PBS in the wells was then transferred to the above tube to collect the cells remaining in the wells in the tube. This tube was centrifuged for 5 min under conditions of 210×g at 4°C. After centrifugation the supernatant was removed, and the precipitate (cell pellet) was suspended (washed) in 1 mL of PBS and centrifuged under the same conditions as described above. This operation was repeated twice, and the supernatant was thereafter removed to obtain cells (a cell pellet) that had been cultured in medium including Sample 1.

The cell penetrating ability of Sample 1 was analyzed by using flow cytometry with respect to the cells (cell pellet) obtained above. An On-chip Flow cytometer (manufactured by On-chip Biotechnologies Co., Ltd.) was used as the flow cytometer.

For this analysis, the cell pellet obtained above was suspended in 50 µL of PBS. A total of 50 µL of 2× sample buffer for flow cytometry was further added to this suspension to prepare a cell suspension for analysis.

Gating was performed using the flow cytometer on the basis of forward scatter (FSC) and side scatter (SSC), a gate was set for the cell population under analysis, and fluorescent intensity was measured for the cell population within this gate. Analysis was performed so that the cell population was composed of about 10,000 cells. Fluorescent intensity was measured using the fluorescence detector FL2 (optimal detection wavelength close to 543 nm) of the flow cytometer, which can detect the fluorescent wavelength of FAM. These measurement results were analyzed using commercial analysis software "FlowJo (registered trademark)" (manufactured by TreeStar Co.) to obtain the mean fluorescent intensity (MFI) of the measured cell population.

### (Examples 2-3)

The operations were the same as in Example 1, except that sample solution 1 was used as the sample solution for each example.

### (Example 4)

The operations were the same as in Example 1, except that a FAM solution diluted with DMSO was used instead of sample solution 1. The concentration of this FAM solution was the same as the concentration of sample solution 1 (that is, the culture solution in the wells had a FAM concentration of 10 µM and a DMSO concentration of 0.5%).

The results obtained for Examples 1 to 4 are shown in Fig. 1 and Table 3. Fig. 1 shows a histogram of fluorescence intensity (horizontal axis) and cell count (vertical axis) measured by the fluorescence detector of the flow cytometer. Table 3 shows the MFI values for each example obtained by such measurements.

### [Table 3]

**Table 3**

| | Example 1 | Example 2 | Example 3 | Example 4 |
|---|---|---|---|---|
| MFI | 1591 | 715 | 489 | 14.3 |

As shown in Fig. 1, in Examples 1 to 3, the histograms shifted to the right on the horizontal axis in comparison with Example 4 in which only FAM was added. As a result, it is understood that any sequence of peptides 1 to 3 has cell membrane penetrating ability and can introduce the foreign substance into at least the cytoplasm. Furthermore, when comparing Examples 1 to 3, in Example 1 using peptide 1 in which the arginine residue of peptide 2 was substituted with a lysine residue, the MFI value was more than twice the MFI value of example 2. Therefore, it is understood that particularly excellent cell membrane penetrating ability can be exhibited. Meanwhile, in Example 3 using peptide 3 in which the lysine residue of peptide 2 was substituted with an arginine residue, the MFI value was greatly reduced compared to Example 2. From these results, it is understood that conservative replacement of basic amino acids (lysine, arginine) contained in the amino acid sequence of peptide 2 greatly affects cell membrane penetrating ability.

Further, although detailed data are not shown, the inventors' research has confirmed that even if the foreign substance is a polypeptide, a nucleic acid or a drug rather than a fluorescent dye, this foreign substance can be introduced efficiently from outside the cell into at least the cytoplasm.

As is clear from the above, with the technique disclosed herein a target foreign substance can be efficiently introduced from outside a eukaryotic cell into at least the cytoplasm of the cell by using a peptide fragment obtained by arranging in series two or more amino acid sequences shown in SEQ ID NO: 1 as a carrier peptide fragment.

Specific examples of the technique disclosed herein have been described in detail above, but these are merely examples and do not limit the scope of the claims. The technique set forth in the claims includes various modifications and changes of the specific examples illustrated above.

### Industrial Applicability

The technique disclosed herein provides an artificially prepared peptide fragment, as well as a construct provided with the peptide fragment, for introducing a target foreign substance from outside a eukaryotic cell into at least the cytoplasm of the cell (in particular, various animal cells such as human and other mammalian cells lacking cell walls). Using this construct, the target foreign substance can be introduced effectively into a target cell to obtain a cell having the introduced foreign substance, and a biological tissue of an organ or the like including the cell having the foreign substance. This construct can also be used to provide therapeutic agents for diseases.

### (Sequence Listing Free Text)

SEQ ID NOS: 1-6 Synthetic peptides

### [Sequence list]

TG22-001PCT_ST25.txt

## Claims

1. A peptide fragment for introducing a target foreign substance from outside a eukaryotic cell into at least the cytoplasm of the cell, the peptide fragment being configured such that two or more of following amino acid sequences
VVKSLVK (SEQ ID NO: 1)
are arranged in series, and another amino acid residue is not included between the amino acid sequences or a peptide linker composed of 1 to 5 amino acid residues is interposed between the amino acid sequences.

2. The peptide fragment according to claim 1, wherein another amino acid residue is not included between the amino acid sequences.

3. The peptide fragment according to claim 1 or 2, wherein two amino acid sequences shown in SEQ ID NO: 1 are arranged in series.

4. A foreign substance introduction construct produced for introducing a target foreign substance from outside a eukaryotic cell into at least the cytoplasm of the cell, the foreign substance introduction construct comprising:
a carrier peptide fragment composed of the peptide fragment according to any one of claims 1 to 3, and
the target foreign substance linked to the N-terminal side and/or C-terminal side of the carrier peptide fragment.

5. The construct according to claim 4, wherein the foreign substance is any organic compound selected from the group consisting of polypeptides, nucleic acids, dyes and drugs.

6. The construct according to claim 5, wherein the foreign substance is a mature polypeptide derived from any species or precursor polypeptide thereof, and the construct is a synthetic polypeptide having an amino acid sequence corresponding to the mature polypeptide or precursor polypeptide thereof as the foreign substance, together with an amino acid sequence of the carrier peptide fragment.

7. The construct according to claim 6, wherein the amino acid sequence corresponding to the mature polypeptide or precursor polypeptide thereof as the foreign substance is arranged on the N-terminal side of the carrier peptide fragment.

8. A method for introducing a target foreign substance from outside a eukaryotic cell into at least the cytoplasm of the cell in vitro, the method including:
(1) a step of preparing the foreign substance introduction construct according to any one of claims 4 to 7;
(2) a step of supplying the foreign substance introduction construct to a sample including a target eukaryotic cell; and
(3) a step of incubating the sample, to which with the foreign substance introduction construct has been supplied, to thereby introduce the foreign substance introduction construct into the eukaryotic cell in the sample.

9. The method according to claim 8, wherein the foreign substance is any organic compound selected from the group consisting of polypeptides, nucleic acids, dyes and drugs.

10. The method according to claim 9, wherein the foreign substance is a mature polypeptide derived from any species or precursor polypeptide thereof, and the foreign matter introduction construct is a synthetic polypeptide having an amino acid sequence corresponding to the mature polypeptide or precursor polypeptide thereof as the foreign substance, together with an amino acid sequence of the carrier peptide fragment.

11. The method according to claim 10, wherein the amino acid sequence corresponding to the mature polypeptide or precursor polypeptide thereof as the foreign substance is arranged on the N-terminal side of the carrier peptide fragment.

12. The method according to any one of claims 8 to 11, wherein the eukaryotic cell of interest into which the foreign substance introduction construct is to be introduced is a human or non-human mammalian cell.
